# EUROPÄISCHE PATENTSCHRIFT

(11) **EP 2 730 296 B1**
(45) Veröffentlichungstag und Bekanntmachung des Hinweises auf die Patenterteilung: **11.01.2017**
(21) Anmeldenummer: 13192578.6
(22) Anmeldetag: 12.11.2013
(51) Int. Cl.: A61L 27/16, A61L 24/06

(54) **Polymethylmethacrylat-Knochenzement**
Polymethyl methacrylate bone cement
Ciment osseux en polyméthylméthacrylate

(30) Priorität: 13.11.2012 DE 102012022134
(43) Veröffentlichungstag der Anmeldung: 14.05.2014
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A1- 2 492 289
- EP-A2- 1 741 455
- WO-A1-2011/141889
- DE-A1-102010 024 653
- DE-T2- 60 100 949
- US-A1- 2006 030 637

## Beschreibung

Gegenstand der Erfindung ist eine härtbare Zusammensetzung zur Verwendung als Knochenzement, insbesondere für die Augmentation von osteoporotischem Knochengewebe, der mindestens ein organisches Polymer und mindestens ein radikalisch polymerisierbares Monomer, 0,02 - 0,14% Gewichtsprozent in Bezug auf die gesamte Zusammensetzung mindestens ein partikuläres, anorganisches Additiv mit einer BET-Oberfläche von mindestens 40 m²/g umfasst, wobei das Additiv kovalent gebundene Hydroxylgruppen aufweist, wobei die Zusammensetzung ferner 1,0-6,0% Gewichtsprozent in Bezug auf die gesamte Zusammensetzung mindestens einen Fettsäureester oder ein Gemisch von Fettsäureestern umfasst, wobei der zumindest eine Fettsäureester oder das Gemisch von Fettsäureestern bei Raumtemperatur (18-25°C) flüssig ist. Ebenfalls Gegenstand der Erfindung ist ein Kit zur Herstellung der Zusammensetzung. Die Zusammensetzung findet zur Augmentation von osteoporotischem Knochengewebe und ganz besonders bevorzugt für die Vertebroplastie, Kyphoplastie und die Augmentation von Bohrlöchern in osteoporotischem Knochengewebe Verwendung. Die erfindungsgemäße Zusammensetzung ist vorzugsweise ein Polymethylmethacrylat-Zement. Polymethylmethacrylat-Knochenzemente (PMMA-Knochenzemente) sind seit Jahrzehnten bekannt und gehen auf die grundlegenden Arbeiten von Sir Charnley zurück (Charnley, J.: "Anchorage of the femoral head prosthesis of the shaft of the femue"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (zum Beispiel N,N-Dimethyl-p-toluidin). Die Pulverkomponente umfasst ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, einen Röntgenopaker und einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Wirbelkörperfrakturen treten häufig bei älteren Patienten mit Osteoporose auf. Diese Wirbelfrakturen verursachen zum Teil erhebliche Schmerzen infolge von Nervenkompressionen. Von Galibert et al. wurden 1987 erstmals die Behandlung von Wirbelkörpern durch Augmentation mit Knochenzement unter dem Begriff "perkutane Vertebroplastie" beschrieben (Galibert P., Deramond H., Rosat P., Le-Gars D. (1987) Note preliminaire sur le traitement des angiomes vertebraux par vertebroplastie acrylique percutanee. [Preliminary note on the treatment of vertebral angioma by percutaneous acrylic vertebroplasty] Neurochirurgie 33: 166-168.) Später wurden weitere Verfahren zur Augmentation von frakturierten Wirbelkörpern entwickelt, wobei die Kyphoplastie die bisher größte Verbreitung fand. Dabei wird zuerst der frakturierte Wirbelkörper mit Hilfe eines Ballons aufgerichtet und danach wird der Wirbelkörper mit Zement augmentiert.

Gegenwärtig wird zur Augmentation von osteoporotischen Wirbelkörpern neben anorganischen Calciumphosphatzementen im Allgemeinen PMMA-Knochenzement eingesetzt. Dabei kommen häufig niedrigviskose PMMA-Zemente zu Anwendung, weil diese leichter appliziert werden können. Die PMMA-Zemente werden dabei mit Hilfe von Spritzen, Schraubsystemen oder Hydrauliksystemen unter Anwendung von relativ hohen Drücken in die Wirbelkörper eingespritzt. Dabei kann es zu ernsten Komplikationen kommen, wenn der Zement aus dem Implantationsgebiet herausfließt und in die ableitenden Venen, in den paravertebralen Raum oder auch in den Spinalkanal eindringt.

Es wurden eine Reihe von Entwicklungen bekannt, die eine Verminderung dieser Risiken bewirken sollen.

Im WO2011117519 wird ein Knochenzement offengelegt, der aus einer Pulverkomponente und einer gelartigen Monomerkomponente zusammengesetzt ist. Die Pulverkomponente enthält ein im Monomer quellbares Polymer, einen Initiator und Röntgenopaker. Die gelartige Komponente ist aus dem Monomer, einem im Monomer gelösten Polymer mit einer Molmasse größer 1.000.000 Dalton und einem Beschleuniger zusammengesetzt. Der Zementteig hat unmittelbar nach Vermischung der Pulverkomponente und der gelartigen Monomerkomponente auf Grund der Verdickerwirkung des in der gelartigen Monomerkomponente gelösten Polymers eine Konsistenz ähnlich eines hochviskosen PMMA-Knochenzementes. Es kann erwartet werden, dass ein hochviskoser Zementteig nur eine geringe Neigung zum Fließen hat und dadurch die Gefahr des unerwünschten Zementaustritts aus den Wirbelkörpern vermindert wird.

Ein anderes Konzept wird im WO2008/032322 offenbart. Hierbei wird eine hohe Viskosität des Zementteigs unmittelbar nach Vermischung der Pulverkomponente mit der flüssigen Monomerkomponente dadurch erreicht, dass im Zementpulver zwei Polymere mit unterschiedlichen Molmassen enthalten sind. Ein erstes Polymer hat eine massemittlere Molmasse (MW) von 150.000-300.000 Dalton und ein zweites Polymer hat eine massemittlere Molmasse (MW) von größer 3.000.000 Dalton. Das niedermolekulare Polymer quillt beziehungsweise löst sich schneller in der Monomerflüsigkeit als das hochmolekulare Polymer. Dadurch wirkt das niedermolekulare Polymer als Verdicker. Der gebildete Zementteig ist dadurch viskos.

Ein besonders interessanter Weg zur Vermeidung von Zementaustritten aus Wirbelkörpern wird im WO2012058305 beschrieben. Hierbei wird ein Dichtungsmittel in den Wirbelkörper eingebracht, dass mit Calciumionen, die immer im Knochengewebe vorhanden sind, unter Ausbildung einer dichtendeten Schicht reagieren und damit alle Spalten und anderen Öffnungen in Wirbelkörpern abdichten. Als Dichtungsmittel wird Natriumalginat vorgeschlagen. Natriumalginat reagiert mit Calciumionen unter Gelbildung.

In EP 2 492 289 A1 wird eine polymerisierbare Zusammensetzung, insbesondere zur Befestigung von Zähnen, beschrieben. Die Zusammensetzung kann neben Acrylblockpolymeren, Monomeren und Initiatoren Weichmacher, wie Paraffin, Erdnussöl und Harzen, oberflächenbehandelte anorganische Füllstoffe enthalten.

WO2011/141889 A1 beschreibt injizierbare Knochenzemente unter anderem für Vertebroplastie und Kyphoplastie, die als Füllstoffe bioaktive Gläser mit 60 bis 85 Mol% SiO₂, 10 bis 30 Mol% CaO und 2 bis 10 Mol% P₂O₅ enthalten.

In DE 601 00 949 T2 wird ferner ein injizierbares Knochenersatzmaterial beschrieben, welches Knochenzement und Öl enthält.

Die Erhöhung der Viskosität der zur Augmentation eingesetzten PMMA-Zemente ist sicher ein Weg, um die Gefahr des unerwünschten Zementaustritts zu vermindern. Aber mit steigender Viskosität des Zementteigs erhöht sich naturgemäß auch der Fließwiderstand bei der Einbringung des Zements durch Kanülen. Das bedeutet, es sind für diese Zemente Applikationssysteme, wie Hydrauliksysteme notwendig, die sehr hohe Drücke erzeugen können, um den Zementteig durch die Kanülen pressen zu können.

Aufgabe der Erfindung war es, eine Zusammensetzung zur Verwendung als Knochenzement, insbesondere einen PMMA-Zement für die Vertebroplastie und Kyphoplastie, zu entwickeln, der möglichst unmittelbar nach der Vermischung der Pulverkomponente mit der Monomerflüssigkeit tropffrei ist. Ebenso sollte eine Zusammensetzung zur Verwendung als Knochenzement bereitgestellt werden die während der Verarbeitungsphase thixotrop ist und eine verminderte Adhäsion an Oberflächen zeigt, insbesondere soll die härtbare Zusammensetzung ein vermindertes Benetzungsverhalten auf Oberflächen zeigen. Eine weitere Aufgabe bestand darin eine Zusammensetzung zur Verwendung als Knochenzement bereitzustellen, die eine verlängerte Verarbeitungsphase nach dem Vermischen der Komponenten hat. Die Zusammensetzung zur Verwendung als Knochenzement, auch als Zementteig bezeichnet, soll möglichst nur bei mechanischer Beanspruchung fließfähig sein. Ohne mechanische Beanspruchung soll die härtbare Zusammensetzung dagegen nur eine geringe Tendenz zum Fließen haben. Weiterhin soll die Zusammensetzung, insbesondere als PMMA-Zement, an Stahl- oder anderen Metalloberflächen nur gering anhaften, um dadurch das Injizieren des Zementes durch Metallkanülen zu erleichtern. Vorteil des thixotropen Verhaltens der härtbaren Zusammensetzung ist einerseits die erleichterte Einbringung der Zusammensetzung unter Schereinwirkung in einer Kanüle unter Druck und andererseits die Verminderung eines unerwünschten Zementaustritts nach der Einbringung der Zusammensetzung in die zu verfüllende Kavität ohne Schereinwirkung. Die Aufgabe der Erfindung wurde gemäß dem Gegenstand des Anspruchs 1 gelöst, bevorzugte Ausführungsformen sind in den Unteransprüchen und detailliert in der Beschreibung erläutert. Gemäß einer Ausführungsform ist die erfindungsgemäße Zusammensetzung ein Polymethylmethacrylat-Zement, umfassend eine Pulverkomponente und eine flüssige Monomerkomponente, wobei die Zusammensetzung 0,02 - 0,14 % Gewichtsprozent in Bezug auf die gesamte Zusammensetzung mindestens ein partikuläres, anorganisches Additiv mit einer BET-Oberfläche von mindestens 40 m²/g umfasst, das an den Partikeln kovalent gebundene Hydroxylgruppen aufweist, und zusätzlich 1,0-6,0 % Gewichtsprozent in Bezug auf die gesamte Zusammensetzung mindestens einen bei Raumtemperatur (18-25°C) flüssigen

Fettsäureester enthält.

Grundidee der Erfindung ist es, ein thixotropierend wirkendes Additiv und ein antiadhäsives, biokompatibles und/oder resorbierbares Additiv einer Zusammensetzung zur Verwendung als Knochenzement zuzusetzen. In diesem Zusammenhang werden vorliegend Zusammensetzung und Knochenzement synonym verwendet, Überraschend war dabei, dass die pastöse Zusammensetzung in Form eines Zementteiges unmittelbar nach der Vermischung der Pulverkomponente mit der flüssigen Monomerkomponente tropffrei ist, während der Verarbeitungsphase nur bei mechanischer Beanspruchung fließfähig ist und ohne mechanische Beanspruchung nur eine geringe Tendenz zum Fließen zeigt.

Ebenso überraschend wurde gefunden, dass durch Zusatz von bei Raumtemperatur flüssigen Fettsäureestern als Komponente der Zusammensetzung zur Verwendung als Knochenzement der erhaltene Zementteig eine im Vergleich zur nicht modifizierten härtbaren Zusammensetzung in Form eines Zementteiges geringere Anhaftung an Metalloberflächen zeigt. Überraschend war weiterhin, dass beide Additive miteinander im Zement verträglich sind. Gegenstand der Erfindung ist eine Zusammensetzung zur Verwendung als Knochenzement umfassend mindestens ein organisches Polymer und mindestens ein polymerisierbares Monomer, wobei die Zusammensetzung härtbar ist und
- mindestens ein radikalisch polymerisierbares Monomer,
- 0,02-0,14 % Gewichtsprozent in Bezug auf die gesamte Zusammensetzung mindestens ein partikuläres, anorganisches Additiv mit einer BET-Oberfläche von mindestens 40 m²/g, wobei das Additiv kovalent gebundene Hydroxylgruppen aufweist, und
- 1,0-6,0 % Gewichtsprozent in Bezug auf die gesamte Zusammensetzung mindestens einen Fettsäureester oder ein Gemisch von Fettsäureestern umfasst,
wobei der zumindest eine Fettsäureester oder das Gemisch von Fettsäureestern bei Raumtemperatur (18-25°C) flüssig ist.

Erfindungsgemäß wird als organisches Polymer ein in dem Monomer lösliches Polymer eingesetzt. Die erfindungsgemäße härtbare Zusammensetzung wird auch synonym als Zementteig, Zement oder Knochenzement bezeichnet. Gemäß einer besonders bevorzugten Ausführungsform ist die Zusammensetzung ein thixotropes und härtbares Fluid oder eine thixotrope und härbare Paste. Als Gemisch von Fettsäureestern gilt vorzugsweise auch ein Neutralöl, MCT-ÖI (CAS-Nummer 73398-61-5), eine Mischung aus mittelkettigen Fettsäuren (Triglyceriden), exakt Caprinsäure und Caprylsäure natürlichen Ursprungs
Um den erfindungsgemäßen Effekt bezüglich der verminderten Anhaftung an vorzugsweise metallischen oder Kunststoffoberflächen zu erzielen und gleichzeitig eine vorzugsweise nur unter erhöhtem Druck fließfähige Zusammensetzung zu erhalten, musste eine Kombination an Additiven gefunden werden, mit denen die gewünschten Eigenschaften gezielt einstellbar sind.

Überraschend wurde gefunden, dass der Zusatz eines Gehaltes von 1,0 - 6,0 % Gewichtsprozent in Bezug auf die gesamte Zusammensetzung mindestens eines Fettsäureesters oder eines Gemisches von Fettsäureestern , wobei der zumindest eine Fettsäureester oder das Gemisch von Fettsäureestern bei Raumtemperatur (18-25°C) flüssig ist, zu einer Knochenzementzusammensetzung die Anhaftung der Zusammensetzung an Oberflächen während der Verarbeitungsphase deutlich vermindert und zugleich keine negativen Effekte auf die Härtung der Zusammensetzung nach ihrer Verbringung in die Knochenkavität hat. Das anorganische Additiv entfaltet seine Wirkung in der Zusammensetzung während der Herstellphase der Zusammensetzung, während der Phase der Verarbeitung als auch in der Phase der Härtung. Das anorganische Additiv muss dabei eine Reihe von Anforderungen erfüllen, es muss zusammen mit den weiteren Komponenten der Zusammensetzung insbesondere den flüssigen Komponenten, wie den Fettsäureestern und/oder den Monomeren schnell eine pastöse, vorzugsweise unter Normaldruck nicht mehr fließfähige Paste bilden, die unter deutlich erhöhtem Druck während der Ausbringung durch eine Kanüle einer Spritze während der Verarbeitungsphase fließfähig wird, und sich die Viskosität der Zusammensetzung unter Druck deutlich vermindert. Nach einer Druckentlastung, im Wesentlichen auf Normaldruck (etwa 1013,25 hPa), insbesondere nach dem Ausbringen der Zusammensetzung in eine Knochenkavität soll die härtbare Zusammensetzung erneut eine erhöhte Viskosität annehmen und während der Aushärtung nicht fließfähig sein. Durch die erfindungsgemäße Kombination von Fettsäureestern und 0,02 - 0,14 % Gewichtsprozent in Bezug auf die gesamte Zusammensetzung anorganischen Additiven mit HO-Gruppen mit einer definierten BET-Oberfläche ist es gelungen eine Zusammensetzung bereitzustellen, die die genannten Anforderungen erfüllt.

Überraschend war weiterhin, dass die beiden zugesetzten Additive die mechanischen Eigenschaften der ausgehärteten Zusammensetzung, d.h. des ausgehärteten PMMA-Zements im Vergleich zu einem konventionellen PMMA-Zement nur minimal beeinflussen und dass es dadurch möglich ist, einen PMMA-Zement zu erhalten, der den Anforderungen der ISO583 hinsichtlich der mechanischen Anforderungen genügt.

Der erfindungsgemäße mindestens eine Fettsäureester oder das Gemisch von Fettsäureestern umfasst vorzugsweise (i) einen Ester aus der Umsetzung mindestens einer Fettsäure mit einem Monoalkohol, Diol, Triol oder Polyol jeweils mit 1 bis 15 C-Atomen, insbesondere mit 1 bis 4 C-Atomen, besonders bevorzugt mit C1 bis C4 Alkyl-Gruppen, oder einem Polyetherpolyol, (ii) einem natürlich vorkommenden Fettsäureester oder einem Fettsäureester natürlicher Herkunft und (iii) einer Mischung enthaltend Fettsäureester aus (i) und (ii). Dabei ist der mindestens eine Fettsäureester oder das Gemisch von Fettsäureestern bei Raumtemperatur zwischen 18 - 25°C flüssig (unter Normaldruck). Als Fettsäureester gelten Fettsäureester, die mindestens eine Fettsäureestergruppe aufweisen bspw. einer Ölsäure

Als besonders bevorzugte Fettsäureester oder Gemische von Fettsäureestern haben sich Fettsäurealkylester erwiesen, insbesondere ein Fettsäurealkylester mit 1 bis 15 C-Atomen in der Alkyl-Gruppe des Esters, bevorzugt mit 1 bis 4 C-Atomen oder 3 C-Atomen des Glycerins, und vorzugsweise eine Fettsäure mit 1 bis 30 C-Atomen in der dem Fettsäurerest, insbesondere mit 1 bis 20 C-Atomen, vorzugsweise 3 bis 20 C-Atomen, wobei gesättigte Fettsäuren bevorzugt sind. Als ebenso bevorzugte Fettsäureester oder Gemische von Fettsäureestern haben sich Fettsäurealkylester, mindestens ein Triglycerid oder eine Mischung enthaltend mindestens eine der genannten Verbindungen erwiesen. Dabei können die Fettsäureester oder das Gemisch von Fettsäureestern generell gesättigte Fettsäuren und/oder ungesättigte Fettsäuren umfassen, wobei sich die gesättigten Fettsäureester als bevorzugt erwiesen haben.

Bevorzugte Fettsäureester umfassen Fettsäuremethylester (FAME) aus pflanzlichen Fetten, denn sie sind üblicherweise bei Raumtemperatur und Normaldruck flüssig. Ebenfalls geeignet sind auch Triacylglycerine mit drei Fettsäuren. Ein bekanntes Triglycerid ist Kokosöl, dass hauptsächlich aus Triglyceriden besteht und gesättigte Fettsäure-Reste enthält, die sich von Capryl-, Laurin-, Caprin-, Palmitin-, Stearin- und Myristinsäure ableiten.

Ebenso Gegenstand der Erfindung sind Polymethylmethacrylat-Zement umfassend eine Pulverkomponente und mindestens eine flüssigen Monomerkomponente wobei die Zusammensetzung 0,02 - 0,14 % Gewichtsprozent in Bezug auf die gesamte Zusammensetzung mindestens ein partikuläres, anorganisches Additiv mit einer BET-Oberfläche von mindestens 40 m²/g, das an den Partikeln kovalent gebundene Hydroxylgruppen aufweist, und - 6,0% Gewichtsprozent in Bezug auf die gesamte Zusammensetzung mindestens einen bei Raumtemperatur (18-25°C) flüssigen Fettsäureester oder Gemische von Fettsäureestern enthält.

Zur Vermeidung unerwünschter Diffusionen in den Körper oder Abtragungen wird zudem eine besonders reine Zusammensetzung bereitgestellt, in der das mindestens eine organische Polymer einen Gehalt größer gleich 99,5 Gew.-%, insbesondere größer gleich 99,8 Gew.-%, weiter bevorzugt größer gleich 99,9 Gew.-% in Bezug auf die gesamte Polymerzusammensetzung aufweist, vorzugsweise ist die Verunreinigung mit Fremdverbindungen im Polymer kleiner gleich 0,5 Gew.-%, insbesondere kleiner gleich 0,2 Gew.-% weiter bevorzugt kleiner gleich 0,1 Gew.-%. Dabei ist insbesondere der Gehalt an organischen Lösemitteln kleiner gleich 0,5 Gew.-%. Weiter ist es bevorzugt, wenn auch das mindestens eine polymerisierbare Monomer einen Gehalt größer gleich 99,1 Gew.-%, insbesondere 99,5 Gew.-% in Bezug auf die gesamte Monomerzusammensetzung aufweist, insbesondere größer gleich 99,8 Gew.-%, besonders bevorzugt größer gleich 99,9 Gew.-%, wobei zugleich die Verunreinigung mit Fremdverbindungen im Monomer kleiner gleich 0,5 Gew.-%, insbesondere kleiner gleich 0,2 Gew.-%, bevorzugt kleiner gleich 0,1 Gew.-% ist.

Ebenso relevant ist der Gehalt des mindestens einen partikulären, anorganischen Additivs. Sein Gehalt an Additiv, insbesondere an SiO₂, ist vorzugsweise größer gleich 99,5 Gew.-%, bevorzugt ist die Verunreinigung des Additivs mit Fremdverbindungen kleiner gleich 0,5 Gew.-%. Insbesondere der Gehalt an ggf. zementzersetzend wirkenden Substanzen, wie freien Säuren oder Chloriden sollte aufgrund des langen Verbleibs des Knochenzementes im Körper gering sein, um langlebige Knochenzemente bereitzustellen.

Aus vorgenannten Gründen weist ein bevorzugter Fettsäureester oder ein Gemisch von Fettsäureestern einen Gehalt in Bezug zur Gesamtzusammensetzung an Fettsäureestern von größer gleich 99,5 Gew.-% auf, vorzugsweise beträgt die Verunreinigung mit Fremdverbindungen in den Fettsäurestern oder Gemischen dieser kleiner gleich 0,5 Gew.-%.

Erfindungsgemäß geeignete partikuläre anorganische Additive weisen an den Partikeln kovalent gebundene HO-Si-Gruppen (Silanol-Gruppen) auf. Durch diese an der Partikeloberfläche angeordneten Hydroxylgruppen können sich Wasserstoff-brückenbindungen zwischen den Füll-stoffpartikeln ausbilden, die durch Einwirkung von mechanischer oder thermischer Energie reversibel gelöst werden können.

Das partikuläre anorganische Additiv ist ausgewählt aus der Gruppe aus pyrogenem Siliziumdioxid, pyrogenem Metall-Siliziummischoxide, Bentonit, Montmorillonit und einer Mischung enthaltend mindestens zwei der genannten Additive.

Daneben ist es auch möglich, hydrophobiertes pyrogenes Siliziumdioxid einzusetzen. Die Herstellung des hydrophoben Siliziumdioxids kann nach dem Stand der Technik durch Behandlung von pyrogenem Siliziumdioxid mit Dialkyldichlorsilanen (z. B. Dimethyldichlorsilan) erfolgen.

Ein ganz besonders bevorzugter partikulärer, anorganischer Füllstoff ist pyrogenes Siliziumdioxid mit einer BET-Oberfläche von mindestens 40 m²/g, weiter bevorzugt von 200 m²/g und am meisten bevorzugt von 300 m²/g. Derartiges pyrogenes Siliziumdioxid ist unter anderem unter dem Markennamen Aerosil^{®} mit spezifischen BET-Oberflächen von 50 m²/g, 90 m²/g, 200 m²/g und 380 m²/g kommerziell verfügbar.

Als partikuläres anorganisches Additiv ist pyrogenes Siliziumdioxid mit einer BET-Oberfläche von mindestens 200 m²/g bevorzugt. Ebenfalls bevorzugt kann als ein partikuläres anorganisches Additiv pyrogenes Siliziumdioxid mit einer BET-Oberfläche von mindestens 300 m²/g eingesetzt werden. Die erfindungsgemäß geeigneten partikulären anorganischen Additive weisen vorzugsweise Primärteilchen mit einem mittleren Teilchendurchmesser (D50) von 5 bis 9, bevorzugt 6 bis 8 nm und besonders bevorzugt von 7 nm und eine spezifische Oberfläche von 270 bis 330 m²/g auf. Besonders bevorzugt wird als partikuläres anorganisches Additiv ein pyrogenes Siliziumdioxid mit einem Gehalt an SiO₂ von größer gleich 99,8 Gew.-% (des geglühten Additivs) und einem Gehalt an Chloriden sowie Fremdmetalloxiden von kleiner gleich 0,2 Gew.-% in Bezug auf das Additiv eingesetzt. Als Fremdmetalloxide im pyrogenen Siliziumdioxid gelten insbesondere Aluminiumoxid, Eisenoxid und Titandioxid.

Die BET-Messung ist ein Analyseverfahren zur Charakterisierung von Oberflächen von Festkörpern mit Hilfe der Gasadsorption. Die Bestimmungsmethode ist in der DIN ISO 9277:2003-05 (Bestimmung der spezifischen Oberfläche von Feststoffen durch Gasadsorption nach dem BET-Verfahren) beschrieben.

Die bei Raumtemperatur flüssigen Ester der gesättigten Fettsäureester sind insbesondere die Methylester, die Ethylester, die n-Propylester und die Isopropylester sowie die Glyceride der Caprylsäure (Octansäure), der Caprinsäure (Decansäure), der Laurinsäure (Dodecansäure), der Myristinsäure (Tetradecansäure), der Palmitinsäure (Hexadecansäure) und der Stearinsäure (Octadecansäure) sowie deren Gemische als bei Raumtemperatur flüssige Fettsäureester.

Als besonders hervorragend geeignet haben sich die nachfolgend genannten Fettsäureester oder Gemische dieser erwiesen, die bei Raumtemperatur flüssig sind: Glycerin-tri-caprylat (Glycerin-tri-octoat), Glycerin-tri-caprinat (Glycerin-tri-decanoat). Ein weiterer Vorteil dieser Fettsäuren ist ihre hohe Reinheit und ihre gute Verträglichkeit. Gemische dieser Fettsäurester sind handelsüblich unter der Bezeichnung Mygliol® 810 erhältlich. Weiterhin kann Propylenglykoldicaprylat und Propylendicaprat als flüssiger Fettsäureester verwendet werden. Diese Ester sind unter der Bezeichnung Mygliol® 840 handelsüblich. Weiterhin sind ähnliche aufgebaute Neutralöle, die zusätzlich Bernsteinsäure und/oder Ölsäure enthalten auch als flüssige Fettsäureester geeignet. Ebenfalls erhältlich übliche Neutralöle oder MCT-Triglyceride, die sich in der prozentualen Zusammensetzung der beiden Fettsäuren, Caprinsäure (C10:0) und Caprylsäure (C8:0) unterscheiden sind u. a. Mygliol 812® der Firma Sasol, Myritol 312® der Firma Cognis und Tegosoft® CT der Firma Evonik. Alle sind Lipide auf Basis von ca. 50-65 % Caprylsäure (C8:0) und ca. 30-45 % Caprinäure (C10:0); Capronsäure (C6:0), Laurinsäure (C12:0) und Myristinsäure (C14:0) sind in sehr geringen Anteilen vorhanden.

Die erfindungsgemäßen Knochenzemente sind Zusammensetzungen umfassend mindestens ein organisches Polymer oder Mischungen von organischen Polymeren, die insbesondere in den Monomeren löslich sind, wobei die Polymere Polyacrylate sind, insbesondere ist das organische Polymer ausgewählt aus Poly(alkyl-2-acrylsäurealkylester), Poly(aryl-2-acrylsäurealkylester), Poly(arylalkyl-2-acrylsäurealkylester) jeweils unabhängig mit 1 bis 18 C-Atomen in der Alkyl-Gruppe, insbesondere mit 1 bis 4 C-Atomen, jeweils unabhängig mit 6 bis 13 C-Atomen in der Aryl-Gruppe, insbesondere mit 6, 10, 12 oder 13 C-Atomen, jeweils unabhängig mit 6 bis 14 C-Atomen in der Arylalkyl-Gruppe, insbesondere mit 8 bis 12 C-Atomen, und jeweils unabhängig mit 1 bis 10 C-Atomen in der Alkylester-Gruppen, insbesondere mit 1 bis 4 C-Atome, oder eine Mischung umfassend mindestens zwei der genannten Polymere.

Besonders bevorzugt ist das organische Polymer, insbesondere ein in dem Monomer lösliches Polymer, ausgewählt aus der Gruppe Poly(methacrylsäuremethylester), Poly(methacrylsäureethylester), Poly(methylmeth-acrylsäurepropylester), Poly(methacrylsäureisopropylester), Poly(methylmeth-acrylat-co-methyl-acrylat), Poly(styrol-co-methyl-meth-acrylat), Copolymeren der genannten Verbindungen und einer Mischung aus mindestens zwei dieser Polymere, wobei Polymethmethacrylat (PMMA) besonders bevorzugt eingesetzt wird,

Als in dem mindestens einen radikalisch polymerisierbaren Monomer lösliches Polymer wird ein Polymer verstanden, das sich in diesem polymerisierbaren Monomer zu wenigstens 10 g/l, vorzugsweise zu wenigstens 25 g/l, besonders bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse (Mw) von wenigstens 150.000 g/mol, insbesondere mindestens 200.000 g/mol bis größer gleich 5000.000 g/mol. Beispielsweise kann es sich bei dem löslichen Polymer um ein Polymer oder Copolymer eines Methacrylsäureesters handeln. Gemäß einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in der erfindungsgemäßen Zusammensetzung liegt üblicherweise in einem Bereich von 1 bis 85 Gew.-%, bezogen auf das Gesamtgewicht der erfindungsgemäßen Zusammensetzung. Entsprechend kann der Gehalt an Polymer jeweils unabhängig in einer der nachstehenden Pasten A, B und/oder D sowie der Pulverkomponente C von 1 bis 85 Gew.-% in Bezug auf die jeweilige Gesamtzusammensetzung an Paste oder Pulverkomponente betragen.

Als besonders bevorzugtes organisches Polymer wird mindestens ein Poly(methacrylsäuremethylester), (PMMA) und als Monomer Methacrylsäuremethylester (MMA) eingesetzt, wobei auch Gemische dieser mit weiteren Monomeren oder ein Co-Polymer von PMMA eingesetzt werden können.

Als in den Monomeren lösliche Polymere, insbesondere Polyacrylate, werden Polymere mit einem Molekulargewicht (Mw) von vorzugsweise größer gleich 200.000 g/mol zur Herstellung von Pulverkomponenten eingesetzt, bevorzugt sind Molekulargewichte von größer gleich 500.000 g/mol. Zur Verwendung in Pasten können auch Polymere mit einem Molekulargewicht von kleiner gleich 500.000 g/mol eingesetzt werden. Dabei bestimmt sich das geeignete Molekulargewicht einerseits danach, ob eine Paste oder einen Pulverkomponenten hergestellt wird sowie den weiteren Komponenten in der Paste als auch, dadurch dass das Polymer in dem eingesetzten Monomer löslich sein muss.

Die in der Zusammensetzung eingesetzten, radikalisch polymerisierbaren Monomere sind vorzugsweise ausgewählt aus mindestens einem Alkyl-2-acrylsäurealkylester, Aryl-2-acrylsäurealkylester, Arylalkyl-2-acrylsäurealkylester jeweils unabhängig mit 1 bis 18 C-Atomen in der linearen, verzweigten oder cyclischen Alkyl-Gruppe, insbesondere mit 1 bis 4 C-Atomen, jeweils unabhängig mit 6 bis 13 C-Atomen in der Aryl-Gruppe, jeweils unabhängig mit 6 bis 14 C-Atomen in der Arylalkyl-Gruppe, insbesondere mit 8 bis 12 C-Atomen, und jeweils unabhängig mit 1 bis 20 C-Atomen in der Alkylester-Gruppe, vorzugsweise mit 1 bis 10 C-Atomen in der Alkylester-Gruppen, wobei die Alkylester-Gruppe eine lineare, verzweigte oder cyclische Alkyl-Gruppe aufweisen kann, insbesondere mit 1 bis 4 C-Atomen, oder eine Mischung umfassend mindestens zwei der genannten Monomere ist, wobei Methacrylsäuremethylester, ein Methacrylsäureester oder ein Alkylacrylsäuremethylester bevorzugt sind. Besonders bevorzugt ist Methacrylsäure-methylester, wie ein Methacrylat-Monomer, insbesondere ein Methacrylat-Monomer, welches bei einer Temperatur von 25°C und einem Druck von 1.013 hPa flüssig ist. Vorzugsweise handelt es sich bei dem radikalisch polymerisierbaren Monomer nicht um einen von Bisphenol A abgeleiteten Methacrylsäureester.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestern kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält.Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Das erfindungsgemäß eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung des radikalisch polymerisierbaren Monomers einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

Vorzugsweise beinhaltet die erfindungsgemäße Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der erfindungsgemäßen Paste.

Eine erfindungsgemäße Zusammensetzung umfasst neben dem löslichen organischen Polymer, insbesondere Polymethmethacrylat (PPMA), und dem radikalisch polymerisierbaren Monomer, insbesondere Methacrylsäuremethylester, das partikuläre, anorganische Additiv in einer Konzentration von 0,02-0,14 Gew.-% in Bezug auf die Gesamtzusammensetzung und den mindestens einen bei Raumtemperatur (18-25°C) flüssigen Fettsäureester in einer Konzentration von 1,0-6,0 Gew.-% in Bezug auf die Gesamtzusammensetzung. Erfindungsgemäß umfasst der aus der Pulverkomponente und der flüssigen Monomerkomponente gemischte Zementteig das partikuläre, anorganische Additiv in einer Konzentration von 0,02-0,14 Gew.-% und den mindestens einen bei Raumtemperatur (18-25°C) flüssigen Fettsäureester in einer Konzentration von 1,0-6,0 Gew.-% in der Gesamtzusammensetzung. Zusätzlich zu den vorgenannten Komponenten umfasst eine erfindungsgemäße Zusammensetzung einen Röntgenopaker, einen Polymerisationsinitiator und/oder einen Polymerisationsbeschleuniger sowie optional zusätzlich Füllstoffe, die nicht dem Additiv entsprechend und lediglich verdickende Wirkung zeigen.

Gemäß einer Ausführungsform der Erfindung wird ein Kit beansprucht, das eine Paste A und eine Paste B umfasst, wobei die
(a) Paste A
   (a1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (a2) mindestens ein in (a1) lösliches organisches Polymer, insbesondere zu 5 bis 50 Gew.-%, bevorzugt 10 bis 40 Gew.-%, besonders bevorzugt 20 bis 30 Gew.-5, und
   (a3) mindestens einen Polymerisationsinitiator beinhaltet; insbesondere zu 0,1 bis 10 Gew.-%, bevorzugt 0,01 bis 8 Gew.-%, besonders bevorzugt 0,01 bis 5 Gew.-%, sowie optional weitere Bestandteile, wie Röntgenopaker und/oder in (a1) unlöslicher Füllstoff, jeweils bezogen auf das Gesamtgewicht der Paste A enthält, und die
(b) Paste B
   (b1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (b2) mindestens ein in (b1) lösliches organisches Polymer, insbesondere zu 5 bis 50 Gew.-%, bevorzugt 10 bis 40 Gew.-%, besonders bevorzugt 20 bis 30 Gew.-5, und
   (b3) mindestens einen Polymerisationsbeschleuniger beinhaltet; insbesondere zu 0,0005 bis 0,5 Gew.-%, sowie optional weitere Bestandteile, wie Röntgenopaker und/oder in (b1) unlöslicher Füllstoff, jeweils bezogen auf das Gesamtgewicht der Paste B enthält, und wobei mindestens eine der Pasten A und B als Komponente (a4) bzw. (b4) 0,02-0,14 % Gewichtsprozent in Bezug auf die gesamte Zusammensetzung wenigstens ein partikuläres anorganisches Additiv mit einer BET-Oberfläche von mindesten 40 m²/g umfasst, wobei das Additiv kovalent gebundene Hydroxylgruppen aufweist und wobei mindestens eine der Pasten A und B als Komponente (a5) bzw. (b5) 1,0-6,0% Gewichtsprozent in Bezug auf die gesamte Zusammensetzung wenigstens einen Fettsäureester oder ein Gemisch von Fettsäureestern aufweist, wobei der zumindest eine Fettsäureester oder das Gemisch von Fettsäureestern bei Raumtemperatur (18-25°C) flüssig ist. Gemäße einer Alternative kann jede Paste einen Gehalt an Fettsäureester aufweisen.

   Dabei kann jeder der Pasten das partikuläre, anorganische Additiv in einer Konzentration 0,001 bis 2 Gew.-%, insbesondere 0,001 bis 1 Gew.-% enthalten, so dass in der durch Vermischen der Pasten A und B, insbesondere im Verhältnis von annähernd 1 zu 1 mit jeweils plus/minus 0,5, erhältlichen Zusammensetzung 0,02-0,14 Gew.-% Additiv in Bezug auf die Gesamtzusammensetzung vorliegen. Entsprechend kann auch der mindestens eine bei Raumtemperatur (18-25 °C) flüssigen Fettsäureester in einer Konzentration von 0,01 bis 30 Gew.-%, insbesondere 0,01 bis 20 Gew.-% jeweils unabhängig in einer der Pasten vorliegen. Der Gehalt an Fettsäureester in der der durch Vermischen der beiden Pasten A und B, insbesondere im Verhältnis von annähernd 1 zu 1 jeweils plus/minus 0,5, erhältlichen Zusammensetzung ist 1,0-6,0 Gew.-% in Bezug auf die Gesamtzusammensetzung. Entsprechendes gilt für die nachstehende Pulverkomponente C und Monomerkomponente D, wobei der Fettsäureester vorzugsweise nur der Monomerkomponente D in entsprechender Menge zugesetzt wird, dass in der aus C und D erhältlichen Zusammensetzung der mindestens eine Fettsäureester zu 1,0-6,0 Gew.-% in der Gesamtzusammensetzung enthalten ist. Wobei C und D vorzugsweise im Verhältnis von annähernd 2 zu 1 bis 1 zu 2 gemischt werden.
   Als Monomere und Polymere werden die vorstehend definierten in den Pasten A und B eingesetzt.
   Entsprechend einer weiteren Ausführungsform wird ein Kit umfassend eine Pulverkomponente C und eine Monomerkomponente D beansprucht, wobei die
(c) Pulverkomponente C
   (c1) mindestens ein pulverförmiges Polyacrylat, insbesondere zu 1 bis 95 Gew.-%, bevorzugte bis 85 Gew.-%,
   (c2) mindestens einen pulverförmigen Röntgenopaker, insbesondere zu 3 bis 60 Gew.-%, bevorzugt 3 bis 30 Gew.-%, und
   (c3) mindestens einen Polymerisationsinitiator beinhaltet; insbesondere zu 0,1 bis 10
      Gew.-%, bevorzugt 0,01 bis 8 Gew.-%, besonders bevorzugt 0,01 bis 5 Gew.-%, sowie optional weitere Bestandteile, wie Röntgenopaker und/oder in (a1) unlöslicher Füllstoff, jeweils bezogen auf das Gesamtgewicht der Paste A enthält,
      und die
(d) Monomerkomponente D
   (d1) wenigstens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (d2) optional ein in (d1) lösliches organisches Polymer, insbesondere zu 5 bis 50 Gew.-%, bevorzugt 10 bis 40 Gew.-%, besonders bevorzugt 20 bis 30 Gew.-5, und (d3) wenigstens einen Polymerisationsbeschleuniger beinhaltet; insbesondere zu 0,0005 bis 0,5 Gew.-% enthält sowie optional weitere Bestandteile, wie Röntgenopaker und/oder in (d1) unlöslichen Füllstoff, jeweils bezogen auf das Gesamtgewicht der Monomerkomponente D, und wobei mindestens die Pulverkomponente C oder die Monomerkomponente D als Komponente (c4) bzw. (d4) 0,02-0,14 % Gewichtsprozent in Bezug auf die gesamte Zusammensetzung
      wenigstens ein partikuläre anorganisches Additiv mit einer BET-Oberfläche von mindesten 40 m²/g aufweist, und wobei das Additiv kovalent gebundene Hydroxylgruppen aufweist, und wobei mindestens die Monomerkomponente D oder die Pulverkomponente C als Komponente (c5) bzw. (d5) 1,0-6,0 % Gewichtsprozent in Bezug auf die gesamte Zusammensetzung wenigstens einen Fettsäureester oder ein Gemisch von Fettsäureestern aufweist, wobei der zumindest eine Fettsäureester oder das Gemisch von Fettsäureestern bei Raumtemperatur (18-25°C) flüssig ist. Bevorzugt weist die Monomerkomponente D den mindestens einen Fettsäureester oder ein Gemisch von Fettsäureestern auf. Als pulverförmiges Polyacrylat wird ein organisches Polymer in Form eines Pulvers gemäß vorstehender Definition eingesetzt, bevorzugt ist pulverförmiges PMMA. Generell kann das Additiv mit einem Gehalt sowohl in der Pulverkomponente als auch in der Paste vorliegen. Die mindestens eine Fettsäure liegt vorzugsweise in der Paste vor.

Im Falle einer erfindungsgemäßen Zusammensetzung, welche durch die Kombination zweier Pasten A und B oder der Pulverkomponente C und der Monomerkomponente D eines Zweikomponenten-Systems erhalten wurde, beinhaltet diese Zusammensetzung vorzugsweise wenigstens einen Polymerisationsinitiator (der in der einen Paste des Zweikomponenten-Systems enthalten war) und wenigstens einen Polymerisationsbeschleuniger (der in der anderen Paste des Zweikomponenten-Systems enthalten war).

Als Monomere und Polymere werden die vorstehend definierten in den Pasten A und B eingesetzt.

Üblicherweise enthalten die Paste A und/oder B sowie die Pulverkomponente C und/oder die Monomerkomponente D jeweils unabhängig voneinander einen Röntgenopaker.

Die vorgenannten Pasten A und B können in jedem beliebigen Verhältnis miteinander gemischt werden, wobei sich die Verwendung der Pasten A und B in einem Verhältnis von im Wesentlichen 1 zu 1, die miteinander gemischt werden können als bevorzugt erwiesen hat, wobei das Verhältnis zueinander unabhängig plus/minus 50 % schwanken kann.

Die erfindungsgemäßen Zusammensetzungen, Pasten und/oder Pulverkomponenten können mindestens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder wenigstens einen Polymerisationsinitiator, wenigstens einen Polymerisationsbeschleuniger und gegebenenfalls wenigstens einen Co-Polymerisationsbeschleuniger beinhalten.

Im Falle eines Einkomponenten-Systems als erfindungsgemäße Zusammensetzung handelt es sich bei dem Polymerisationsinitiator vorzugsweise um einen aktivierbaren Polymerisationsinitiator, z. B. um einen in der Zusammensetzung, die als Paste vorliegt, gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems die erfindungsgemäße Zusammensetzung oder Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden erfindungsgemäß Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Gemäß einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amylhydroperoxid, Di-Isopropylbenzen-mono-hydroperoxid und einer Mischung aus mindestens zwei davon besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten. Gemäß einer besonderen Ausgestaltung der erfindungsgemäßen Paste ist das Barbitursäurederivat ausgewählt aus der Gruppe bestehend aus 1,5-disubstituierten Barbituraten-und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Erfindungsgemäß bevorzugt sind Barbiturate, die an Position 1 und an Position 5 jeweils einen Substituenten oder an den Positionen 1, 3 und 5 jeweils einen Substituenten -tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt. Erfindungsgemäß bevorzugte Schwermetallverbindungen sind ausgewählt aus der Gruppe bestehend Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der erfindungsgemäßen Zusammensetzung oder Paste ist der Polymerisationsbeschleuniger ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäurediimid und einer Mischung aus mindestens zwei davon.

Eine weitere vorteilhafte Ausgestaltung der Erfindung umfasst eine Verwendung von Kombinationen aus Schwermetallsalzen und mindestens einem Vertreter aus der Gruppe umfassend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung der Erfindung besteht darin, dass gegebenenfalls wenigstens ein Co-Polymerisationsbeschleuniger in der erfindungsgemäßen Zusammensetzung oder in einer der Pasten A, B oder D enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin, 1,8-Diazabicyclo[5.4.0-]undec-7-en und 1,5-Diazabicyclo(4.3.0)-non-5-en als Co-Akzeleratoren bevorzugt sind.

Die erfindungsgemäße Zusammensetzung, insbesondere in Form einer Paste kann den Polymerisationsinitiator, den Polymerisationsbeschleuniger, den Co-Polymerisations-beschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht der erfindungsgemäßen Zusammensetzung oder jeweils unabhängig bezogen auf das Gesamtgewicht einer der Pasten A, B oder D beinhalten.

Die erfindungsgemäße Zusammensetzung, insbesondere in Form einer Paste oder die Pasten A, B oder D als auch die Pulverkomponente C können neben den vorstehend genannten Komponenten weitere Bestandteile beinhalten.

Gemäß einer bevorzugten Ausführungsform der erfindungsgemäßen Zusammensetzung oder einer der Pasten A, B oder D sowie die Pulverkomponente C können diese jeweils unabhängig voneinander mindestens einen Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentrationen an Röntgenopaker, insbesondere die Konzentration an Zirkoniumdioxid, in der erfindungsgemäßen Zusammen-setzung oder einer der Pasten A, B oder D sowie in der Pulverkomponente C können jeweils voneinander unabhängig beispielsweise in einem Bereich von 3 bis 30 Gew.-% in Bezug auf die jeweilige Gesamtzusammensetzung liegen. Röntgenopaker gelten vorliegend nicht als Füllstoffe.

Gemäß einer weiteren bevorzugten Ausführungsform kann die erfindungsgemäße Zusammensetzung oder eine der vorgenannten Pasten mindestens einen Farbstoff beinhalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform kann die erfindungsgemäße Zusammensetzung mindestens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die erfindungsgemäße Zusammensetzung mindestens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die erfindungsgemäße Zusammensetzung oder mindestens eine der Pasten A, B oder D mindestens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Vorzugsweise ist der Kit als Vorrichtung zur Herstellung von Zusammensetzungen zur Verwendung als Knochenzement so ausgestaltet, dass es einen ersten Behälter und einen zweiten Behälter aufweist, wobei der erste Behälter die Paste A und der zweite Behälter die Paste B aufweist, wobei wenigstens einer der Behälter zu öffnen ist, um nach dem Öffnen ein Vermischen der Pasten A und B zu gestatten, und eine Mischeinheit zum Vermischen der Pasten A und B. Entsprechend kann das Kit als Vorrichtung zur Herstellung der erfindungsgemäßen Zusammensetzung einen ersten Behälter für die Pulverkomponente C und die Monomerkomponente D aufweisen.

Im Falle des Kits werden dazu die wenigstens zwei Pasten A und B miteinander vermischt, wobei die erfindungsgemäße Zusammensetzung erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B. Das Mischungsverhältnis der Pulverkomponente C und der Monomerkomponente D beträgt vorzugsweise 3 zu 1 bis 1 zu 2, insbesondere um 2 zu 1 Gewichtsteile. Das Mischen kann mit üblichen Mischgeräten, beispielsweise einem statischen Mischer oder einem dynamischen Mischer erfolgen.

Nach dem Vermischen der Pasten des Kits ist die letztlich erhaltene Zusammensetzung sofort klebfrei (Norm ISO 5833).

Einsetzbar ist die erfindungsgemäße Zusammensetzung innerhalb von 20 bis 60 Sekunden nach dem Herstellen der Zusammensetzung, insbesondere nach Vermischen der Pasten A und B oder Pulverkomponente C und der Monomerkomponente D. Die erfindungsgemäße Zusammensetzung daher sehr schnell ab circa 20 Sekunden nach dem Vermischen bereits tropffrei und einsetzbar. Eine übliche erfindungsgemäße tropffreie Zusammensetzung kann bereits nach etwa 40 Sekunden nach dem Beginn des Vermischens erhalten werden. Die so hergestellte härtbare Zusammensetzung ist dann für etwa 8 bis 9 Minuten verarbeitbar. Das nutzbare Zeitfenster zur Verarbeitung der Zusammensetzung konnte somit auf circa 8 bis 9 Minuten vergrößert werden. Ähnliche Knochenzemente aus dem Stand der Technik werden erst nach 2 bis 3 Minuten nach dem Vermischen tropffrei und können anschließend nur über einen Zeitraum von 7 Minuten verarbeitet werden, wobei sie zudem den Nachteil aufweisen sich aufgrund der Anhaftung schlecht durch eine Kanüle pressen zu lassen. Somit weisen die erfindungsgemäßen Zusammensetzung den Vorteil einer schnellen Einsetzbarkeit und leichteren Applizierbarkeit innerhalb eines größeren Zeitfensters auf.

Der aus der erfindungsgemäßen Paste bzw. der aus Paste C durch Aushärtung entstehende Knochenzement erreicht etwa sechs bis acht Minuten nach dem Vermischen der im Kit enthaltenen Pasten oder Komponenten eine hohe Festigkeit.

Ebenfalls Gegenstand der Erfindung ist ein Knochenzement erhältlich durch Vermischen der Pasten A und B oder der Pulverkomponente C mit der Monomerkomponente D, insbesondere der härtbare Knochenzement und auch der ausgehärtete Knochenzement.

Ebenso Gegenstand der Erfindung ist ein Formkörper erhältlich durch Polymerisation einer erfindungsgemäßen Zusammensetzung durch Vermischen der Pasten A und B oder der Pulverkomponente C mit der Monomerkomponente D und Polymerisation.

Die erfindungsgemäßen Knochenzemente und Zusammensetzungen oder Kits sind dazu geeignet in der Augmentation von osteoporotischen Knochengewebe und ganz besonders bevorzugt in Vertebroplastie oder Kyphoplastie und besonders zur Augmentation von Bohrlöchern in osteoporotischem Knochengeweben zum Einsatz zu kommen. Gleichfalls geeignet sind die Knochenzemente zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für lokale Freisetzung von pharmazeutisch wirksamen Substanzen.

Der erfindungsgemäße Zement kann durch kanülierte Schrauben in das osteoporotische Knochengewebe eingebracht werden und stabilisiert nach seiner Aushärtung den Verbund zwischen dem Knochengewebe und den kanülierten Schrauben. Neben der Vertebroplastie und Kyphoplastie kann der Zement auch bei von diesen Verfahren abgeleiteten Verfahren, wie zum Beispiel der Vesselplastie, eingesetzt werden.

Die Erfindung wird durch nachstehende Beispiele näher erläutert, ohne jedoch die Erfindung zu beschränken.

Es wurden für die Beispiele 1 und 2, das Vergleichsbeispiel und das Referenzbeispiel ein Polymethylmethacrylat-co-methylacrylat (Molmasse > 500.000 g/mol); handelsübliches Dibenzoylperoxid (BPO phlegmatisiert mit 25 Gew.% Wasser), Aerosil® 380 (Evonik) und Glycerin-1,2,3-tric-octoat (Sigma-Aldrich) verwendet.

| Beispiel-Nr. | Zusammensetzung | | | |
|---|---|---|---|---|
| | Polymer [g] | BPO 75%ig [mg] | Aerosil 380 [mg] | Glycerin-1,2,3-tric-octoat [g] |
| 1 | 25,90 | 98 | 50 | 1,00 |
| 2 | 25,90 | 98 | 50 | 1,50 |
| Vergleich | 25,90 | 98 | 50 | 2,00 |
| Referenz | 25,90 | 98 | - | - |

Die Monomerflüssigkeit hatte folgende Zusammensetzung 99,3 Gew.-% Methylmethacrylat und 0,7 Gew.-% N,N-Dimethyl-p-toluidin. Das Methylmethacrylat enthielt als Stabilisator 10 ppm Hydrochinon.

Es wurden die pulverförmigen Zementzusammensetzungen der Beispiele 1-3 und des Referenzbeispiels mit jeweils 10 ml der Monomerflüssigkeit innerhalb von 30 Sekunden vermischt. Es entstand bei den Beispielen 1-3 nach ca. 40 Sekunden ein tropffreier Zementteig, der eine Verarbeitungsphase von ungefähr 8-9 Minuten hatte. In dieser Verarbeitungsphase konnten diese Proben problemlos durch 18 G-Kanülen (Kanüle mit 1,2 mm Außendurchmesser) mit einer 3 ml Spritze gepresst werden. Der Zementteig haftete nur gering an der Kanüle.
Es entstand bei dem Referenzzement erst nach einer Wartezeit von 2-3 Minuten ein tropffreier Zementteig der dann eine Verarbeitungsphase von ungefähr 7 Minuten hatte. Der Zementteig zeigte eine ausgeprägte Haftung an der 18 G-Kanüle und ließ sich recht schwer auspressen.

Mit dem durch Vermischung der Pulverkomponente und der Monomerflüssigkeit hergestellten Zementteig der Beispiele 1-3 und der Referenz wurden streifenförmige Prüfkörper mit den Abmessungen (75 mm × 10 mm × 3,3 mm) für die Bestimmung der Biegefestigkeit und des Bie-gemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

| Proben-Nr. | 4-Punkt-Biegefestigkeit [MPa] | Biegemodul [MPa] | Druckfestigkeit [MPa] |
|---|---|---|---|
| 1 | 60,1 ± 1,2 | 2999 ± 145 | 88,3 ±2,0 |
| 2 | 58,3 ± 1,1 | 2851 ± 102 | 85,7 ± 2,4 |
| Vergleich | 45,6 ± 1,7 | 2174 ± 60 | 86,3 ± 1,9 |
| Referenz | 54,9 ± 0,9 | 2741 ± 57 | 91,7 ± 0,9 |

Die Ergebnisse der Prüfung der 4-Punkt-Biegefestigkeit, des Biegemoduls und der Druckfestigkeit der ausgehärteten Zemente 1 und 2 zeigen, dass die Anforderungen der ISO5833 hinsichtlich der mechanischen Festigkeit erfüllt werden. Beim Beispiel 3 (Vergleich) werden die Anforderungen der ISO5833 bezüglich des Biegemoduls und der Druckfestigkeit erfüllt, jedoch die Anforderungen hinsichtlich der 4-Punkt-Biegefestigkeit nicht. Die ISO5833 schreibt folgende Parameter vor: eine 4-Punkt-Biegefestigkeit von mindestens 50 MPa, ein Biegemodul von mindestens 1800 MPa und eine Druckfestigkeit von mindestens 70 MPa.

## Patentansprüche

1. Zusammensetzung zur Verwendung als Knochenzement umfassend mindestens ein organisches Polymer und mindestens ein polymerisierbares Monomer, **dadurch gekennzeichnet, dass** die Zusammensetzung härtbar ist und
- mindestens ein radikalisch polymerisierbares Monomer,
- 0,02 - 0,14 % Gewichtsprozent in Bezug auf die gesamte Zusammensetzung mindestens eines partikuläres, anorganisches Additiv mit einer BET-Oberfläche von mindestens 40 m²/g, wobei das Additiv kovalent gebundene Hydroxylgruppen aufweist, und
- 1,0-6,0% Gewichtsprozent in Bezug auf die gesamte Zusammensetzung mindestens einen Fettsäureester oder ein Gemisch von Fettsäureestern umfasst, wobei der zumindest eine Fettsäureester oder das Gemisch von Fettsäureestern bei Raumtemperatur (1825°C) flüssig ist.

2. Zusammensetzung nach Anspruch 1, **dadurch gekennzeichnet, dass** die Zusammensetzung ein thixopropes und härtbares Fluid oder eine thioxotrope und härbare Paste ist.

3. Zusammensetzung nach Anspruch 1 oder 2, **dadurch gekennzeichnet, dass** der mindestens eine Fettsäureester oder das Gemisch von Fettsäureestern ausgewählt ist aus
(i) einem Ester aus der Umsetzung mindestens einer Fettsäure mit einem Monoalkohol, Diol, Triol oder Polyol jeweils mit 1 bis 15 C-Atomen oder einem Polyetherpolyol
(ii) einem natürlich vorkommenden Fettsäureester oder einem Fettsäureester natürlicher Herkunft und
(iii) einer Mischung enthaltend Fettsäureester aus (i) und (ii).

4. Zusammensetzung nach einem der Ansprüche 1 bis 3, **dadurch gekennzeichnet, dass** der Fettsäureester oder das Gemisch von Fettsäureestern mindestens einen Fettsäurealkylester, mindestens ein Triglycerid oder eine Mischung enthaltend mindestens eine der genannten Verbindungen umfasst.

5. Zusammensetzung nach einem der Ansprüche 1 bis 4, **dadurch gekennzeichnet, dass** der Fettsäureester oder das Gemisch von Fettsäureestern gesättigte Fettsäuren und/oder ungesättigte Fettsäuren umfasst.

6. Zusammensetzung nach einem der Ansprüche 1 bis 5, **dadurch gekennzeichnet, dass** das partikuläre anorganische Additiv an den Partikeln kovalent gebundene HO-Si-Gruppen (Silanol-Gruppen) aufweist.

7. Zusammensetzung nach einem der Ansprüche 1 bis 6, **dadurch gekennzeichnet, dass** das partikuläre anorganische Additiv ausgewählt ist aus der Gruppe pyrogenes Siliziumdioxid, pyrogenes Metall-Siliziummischoxide, Bentonit, Montmorillonit und einer Mischung enthaltend mindestens zwei der genannten Additive.

8. Zusammensetzung nach einem der Ansprüche 1 bis 7, **dadurch gekennzeichnet, dass** das partikuläre anorganische Additiv ein pyrogenes Siliziumdioxid mit einem Gehalt an SiO₂ von größer gleich 99,8 Gew.-% (des geglühten Additivs) aufweist und der Gehalt an Chloriden sowie Fremdmetalloxiden kleiner gleich 0,2 Gew.-% in Bezug auf das Additiv beträgt.

9. Zusammensetzung nach einem der Ansprüche 1 bis 8, **dadurch gekennzeichnet, dass** das partikuläre anorganische Additiv pyrogenes Siliziumdioxid mit einer BET-Oberfläche von mindestens 200 m²/g ist.

10. Zusammensetzung nach Anspruch 1 oder 9, **dadurch gekennzeichnet, dass** partikuläre anorganische Additiv pyrogenes Siliziumdioxid mit einer BET-Oberfläche von mindestens 300 m²/g ist.

11. Zusammensetzung nach einem der Ansprüche 1 bis 10, **dadurch gekennzeichnet, dass** die bei Raumtemperatur flüssigen Fettsäureester aus Glycerin-tri-caprylat (Glycerin-tri-octoat), Glycerin-tri-caprinat (Glycerin-tri-decanoat), Propylenglykoldicaprylat und Propylendicaprat und Gemischen enthaltend mindestens zwei der genannten Verbindungen ausgewählt sind.

12. Zusammensetzung nach einem der Ansprüche 1 bis 11, **dadurch gekennzeichnet, dass** die bei Raumtemperatur flüssigen Fettsäureester aus die Methylester, die Ethylester, die n-Propylester, die Isopropylester und Glyceride der Caprylsäure (Octansäure), der Caprinsäure (Decansäure), der Laurinsäure (Dodecansäure), der Myristinsäure (Tetradecansäure), der Palmitinsäure (Hexadecansäure) und der Stearinsäure (Octadecansäure) und deren Gemischen ausgewählt sind.

13. Zusammensetzung nach einem der Ansprüche 1 bis 12, **dadurch gekennzeichnet, dass** das organische Polymer ausgewählt ist aus Poly(alkyl-2-acrylsäurealkylester), Poly(aryl-2-acrylsäurealkylester), Poly(arylalkyl-2-acrylsäurealkylester) jeweils unabhängig mit 1 bis 18 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppen oder einer Mischung umfassend mindestens zwei der genannten Polymere.

14. Zusammensetzung nach Anspruche 1 oder 13, **dadurch gekennzeichnet, dass** das organische Polymer mindestens ein Poly(methacrylsäuremethylester), (PMMA) und das Monomer Methacrylsäuremethylester (MMA) umfasst.

15. Zusammensetzung nach einem der Ansprüche 1 oder 14, **dadurch gekennzeichnet, dass** das organische Polymer ausgewählt ist aus der Gruppe Poly(methacrylsäuremethylester), Poly(methacrylsäureethylester), Poly(methylmethacrylsäurepropylester), Poly(methacrylsäureisopropylester), Poly(methylmeth-acrylat-comethyl-acrylat), Poly(styrol-co-methyl-meth-acrylat), Copolymeren der genannten Verbindungen und einer Mischung aus mindestens zwei dieser Polymere.

16. Zusammensetzung nach einem der Ansprüche 1 bis 15, **dadurch gekennzeichnet, dass** das Monomer ausgewählt ist aus mindestens einem Alkyl-2-acrylsäurealkylester, Aryl-2-acrylsäurealkylester, Arylalkyl-2-acrylsäurealkylester jeweils unabhängig mit 1 bis 18 C-Atome in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppen oder eine Mischung umfassend mindestens zwei der genannten Monomere.

17. Kit, aufweisend eine Paste A und eine Paste B, wobei
(a) Paste A
(a1) mindestens ein radikalisch polymerisierbares Monomer,
(a2) mindestens ein in (a1) lösliches organisches Polymer, und
(a3) mindestens einen Polymerisationsinitiator beinhaltet;
(b) Paste B
(b1) mindestens ein radikalisch polymerisierbares Monomer,
(b2) mindestens ein in (b1) lösliches organisches Polymer, und
(b3) mindestens einen Polymerisationsbeschleuniger beinhaltet;
und wobei mindestens eine der Pasten A und B als Komponente (a4) bzw. (b4) wenigstens ein partikuläre anorganisches Additiv mit einer BET-Oberfläche von mindesten 40 m²/g umfasst, wobei das Additiv kovalent gebundene Hydroxylgruppen aufweist und, wobei mindestens eine der Pasten A und B als Komponente (a5) bzw. (b5) wenigstens einen Fettsäureester oder ein Gemisch von Fettsäureestern aufweist, wobei das mindestens eine partikuläre, anorganische Additiv zu 0,02-0,14 % Gewichtsprozent in Bezug auf die gesamte Zusammensetzung vorliegt und der zumindest eine Fettsäureester oder das Gemisch von Fettsäureestern zu 1,0-6,0 % Gewichtsprozent in Bezug auf die gesamte Zusammensetzung vorliegt und der zumindest eine Fettsäureester oder das Gemisch von Fettsäureestern bei Raumtemperatur (18 - 25°C) flüssig ist.

18. Kit, aufweisend eine Pulverkomponente C und Monomerkomponente D, wobei die
(c) Pulverkomponente C
(c1) mindestens ein pulverförmiges Polyacrylat,
(c2) mindestens einen pulverförmigen Röntgenopaker, und
(c3) mindestens einen Polymerisationsinitiator beinhaltet;
(d) Monomerkomponente D
(d1) wenigstens ein radikalisch polymerisierbares Monomer,
(d2) optional ein in (d1) lösliches organisches Polymer, und
(d3) wenigstens einen Polymerisationsbeschleuniger beinhaltet;
und wobei mindestens die Pulverkomponente C oder Monomerkomponente D als Komponente (c4) bzw. (d4) wenigstens ein partikuläre anorganisches Additiv mit einer BET-Oberfläche von mindesten 40 m²/g umfasst, und wobei das Additiv kovalent gebundene Hydroxylgruppen aufweist und, wobei mindestens die Monomerkomponente D oder die Pulverkomponente C als Komponente (c5) bzw. (d5) wenigstens einen Fettsäureester oder ein Gemisch von Fettsäureestern aufweist, wobei das mindestens eine partikuläre, anorganische Additiv zu 0,02 - 0,14 % Gewichtsprozent in Bezug auf die gesamte Zusammensetzung vorliegt und der zumindest eine Fettsäureester oder das Gemisch von Fettsäureestern zu 1,0-6,0 % Gewichtsprozent in Bezug auf die gesamte Zusammensetzung vorliegt und der zumindest eine Fettsäureester oder das Gemisch von Fettsäureestern bei Raumtemperatur (18-25°C) flüssig ist.

19. Härtbarer Knochenzement erhältlich durch Vermischen der in dem Kit gemäß Anspruch 17 enthaltenden Pasten A und B oder der in dem Kit gemäß Anspruch 18 enthaltenden Pulverkomponente C mit der Monomerkomponente D.

20. Formkörper erhältlich durch Polymerisation einer Zusammensetzung nach einem der Ansprüche 1 bis 16 oder durch Vermischen der in dem Kit gemäß Anspruch 17 enthaltenden Pasten A und B oder der in dem Kit gemäß Anspruch 18 enthaltenden Pulverkomponente C mit der Monomerkomponente D und Polymerisation.

## Claims

1. Composition for use as bone cement comprising at least one organic polymer and at least one polymerisable monomer, **characterised in that** the composition can be cured and comprises
- at least one monomer for radical polymerisation;
- 0.02 - 0.14% by weight, relative to the total composition, of at least one particulate inorganic additive having a BET surface of at least 40 m²/g, whereby the additive comprises covalently bound hydroxyl groups; and
- 1.0 - 6.0% by weight, relative to the total composition, of at least one fatty acid ester or a mixture of fatty acid esters, whereby the at least one fatty acid ester or the mixture of fatty acid esters is liquid at room temperature (18-25°C).

2. Composition according to claim 1, **characterised in that** the composition is a thixotropic and curable fluid or a thixotropic and curable paste.

3. Composition according to claim 1 or 2, **characterised in that** the at least one fatty acid ester or the mixture of fatty acid esters is selected from
(i) an ester from the reaction of at least one fatty acid with a mono-alcohol, diol, triol or polyol, each having 1 to 15 C-atoms, or a polyetherpolyol
(ii) a naturally occurring fatty acid ester or a fatty acid ester of natural origin, and
(iii) a mixture containing fatty acid esters from (i) and (ii).

4. Composition according to any one of the claims 1 to 3, **characterised in that** the fatty acid ester or the mixture of fatty acid esters comprises at least one fatty acid alkyl ester, at least one triglyceride or a mixture containing at least one of the aforementioned compounds.

5. Composition according to any one of the claims 1 to 4, **characterised in that** the fatty acid ester or the mixture of fatty acid esters comprises saturated fatty acids and/or unsaturated fatty acids.

6. Composition according to any one of the claims 1 to 5, **characterised in that** the particulate inorganic additive comprises HO-Si groups (silanol groups) covalently bound to the particles.

7. Composition according to any one of the claims 1 to 6, **characterised in that** the particulate inorganic additive is selected from the group of pyrogenic silicon dioxide, pyrogenic metal-silicon mixed oxides, bentonite, montmorillonite, and a mixture containing at least two of the aforementioned additives.

8. Composition according to any one of the claims 1 to 7, **characterised in that** the particulate inorganic additive comprises a pyrogenic silicon dioxide with an SiO₂ content of more than/equal to 99.8% by weight (of the annealed additive) and **in that** the content of chlorides and foreign metal oxides is less than/equal to 0.2% by weight relative to the additive.

9. Composition according to any one of the claims 1 to 8, **characterised in that** the particulate inorganic additive is pyrogenic silicon dioxide having a BET surface of at least 200 m²/g.

10. Composition according to any one of the claims 1 or 9, **characterised in that** the particulate inorganic additive is pyrogenic silicon dioxide having a BET surface of at least 300 m²/g.

11. Composition according to any one of the claims 1 to 10, **characterised in that** the fatty acid esters that are liquid at room temperature are selected from glycerol-tri-caprylate (glycerol-tri-octoate), glycerol-tri-caprinate (glycerol-tridecanoate), propyleneglycol dicaprylate and propylene dicaprate and mixtures containing at least two of the aforementioned compounds.

12. Composition according to any one of the claims 1 to 11, **characterised in that** the fatty acid esters that are liquid at room temperature are selected from the methyl esters, the ethyl esters, the n-propyl esters, the isopropyl esters and glycerides of caprylic acid (octanoic acid), caprinic acid (decanoic acid), lauric acid (dodecanoic acid), myristic acid (tetradecanoic acid), palmitic acid (hexadecanoic acid), and stearic acid (octadecanoic acid), and the mixtures thereof.

13. Composition according to any one of the claims 1 to 12, **characterised in that** the organic polymer is selected from poly(alkyl-2-acrylic acid alkyl ester), poly(aryl-2-acrylic acid alkyl ester), poly(arylalkyl-2-acrylic acid alkyl ester), each independently having 1 to 18 C-atoms in the alkyl group, each independently having 6 to 14 C-atoms in the aryl group, each independently having 6 to 14 C-atoms in the arylalkyl group, and each independently having 1 to 10 C-atoms in the alkyl ester group, or a mixture comprising at least two of said polymers.

14. Composition according to claim 1 or 13, **characterised in that** the organic polymer comprises at least one poly(methacrylic acid methylester) (PMMA) and the methacrylic acid methyl ester (MMA) monomer.

15. Composition according to any one of the claims 1 or 14, **characterised in that** the organic polymer is selected from the group of poly(methacrylic acid methylester), poly(methacrylic acid ethylester), poly(methylmethacrylic acid propylester), poly(methacrylic acid isopropylester), poly(methylmethacrylateco-methylacrylate), poly(styrene-co-methylmethacrylate), copolymers of said compounds, and a mixture of at least two of these polymers.

16. Composition according to any one of the claims 1 to 15, **characterised in that** the monomer is selected from at least one alkyl-2-acrylic acid alkyl ester, aryl-2-acrylic acid alkyl ester, arylalkyl-2-acrylic acid alkyl ester, each independently having 1 to 18 C-atoms in the alkyl group, each independently having 6 to 14 C-atoms in the aryl group, each independently having 6 to 14 C-atoms in the arylalkyl group, and each independently having 1 to 10 C-atoms in the alkyl ester group, or a mixture comprising at least two of said monomers.

17. Kit comprising a paste A and a paste B, whereby
(a) paste A contains
(a1) at least one monomer for radical polymerisation;
(a2) at least one organic polymer that is soluble in (a1); and
(a3) at least one polymerisation initiator;
(b) paste B contains
(b1) at least one monomer for radical polymerisation;
(b2) at least one organic polymer that is soluble in (b1); and
(b3) at least one polymerisation accelerator;
and whereby at least one of the pastes A and B comprises, as component (a4) and/or (b4), at least one particulate inorganic additive having a BET surface of at least 40 m²/g, whereby the additive comprises covalently bound hydroxyl groups, whereby at least one of the pastes A and B comprises, as component (a5) and/or (b5), at least one fatty acid ester or a mixture of fatty acid esters, whereby 0.02 - 0.14% by weight of the at least one particulate inorganic additive, relative to the total composition, are present and 1.0 - 6.0% by weight of the at least one fatty acid ester or the mixture of fatty acid esters, relative to the total composition, are present, and the at least one fatty acid ester or the mixture of fatty acid esters is liquid at room temperature (18-25°C).

18. Kit comprising a powder component C and monomer component D, whereby the
(c) powder component C contains
(c1) at least one powdered polyacrylate;
(c2) at least one powdered radiopaquer; and
(c3) at least one polymerisation initiator;
(d) monomer component D contains
(d1) at least one monomer for radical polymerisation;
(d2) optionally, an organic polymer that is soluble in (d1); and
(d3) at least one polymerisation accelerator;
and whereby at least powder component C or monomer component D comprises, as component (c4) and/or (d4), at least one particulate inorganic additive having a BET surface of at least 40 m²/g, and whereby the additive comprises covalently bound hydroxyl groups, whereby at least powder component C or monomer component D comprises, as component (c5) and/or (d5), at least one fatty acid ester or a mixture of fatty acid esters, whereby 0.02 - 0.14% by weight of the at least one particulate inorganic additive, relative to the total composition, are present and 1.0 - 6.0% by weight of the at least one fatty acid ester or the mixture of fatty acid esters, relative to the total composition, are present, and the at least one fatty acid ester or the mixture of fatty acid esters is liquid at room temperature (18-25°C).

19. Curable bone cement obtainable by mixing the pastes A and B contained in the kit according to claim 17 or the powder component C and monomer component D contained in the kit according to claim 18.

20. Form body obtainable by polymerisation of a composition according to any one of the claims 1 to 16 or by mixing the pastes A and B contained in the kit according to claim 17 or the powder component C and monomer component D contained in the kit according to claim 18 and polymerisation.

## Revendications

1. Composition pour l'utilisation en tant que ciment osseux comprenant au moins un polymère organique et au moins un monomère polymérisable, **caractérisée en ce que** la composition peut être durcie et comprend
- au moins un monomère polymérisable par voie radicalaire,
- 0,02 à 0,14 % pourcent en poids par rapport à la composition totale d'au moins un additif particulaire inorganique avec une surface spécifique d'au moins 40 m²/g, dans laquelle l'additif présente des groupes hydroxyle liés par covalence, et
- 1,0 à 6,0 % pourcent en poids par rapport à la composition totale d'au moins un ester d'acide gras ou d'un mélange d'esters d'acide gras, dans laquelle l'au moins un ester d'acide gras ou le mélange d'esters d'acide gras est fluide à température ambiante (18 à 25°C).

2. Composition selon la revendication 1, **caractérisée en ce que** la composition est un fluide thixotrope et durcissable ou une pâte thixotrope et durcissable.

3. Composition selon la revendication 1 ou 2, **caractérisée en ce que** l'au moins un ester d'acide gras ou le mélange d'esters d'acide gras est sélectionné parmi
(i) un ester provenant de la transformation d'au moins un acide gras avec un monoalcool, diol, triol ou polyol à chaque fois avec 1 à 15 atomes de C ou un polyétherpolyol
(ii) un ester d'acide gras se produisant naturellement ou un ester d'acide gras de provenance naturelle et
(iii) un mélange contenant des esters d'acide gras de (i) et (ii).

4. Composition selon une des revendications 1 à 3, **caractérisée en ce que** l'ester d'acide gras ou le mélange d'esters d'acide gras comprend au moins un ester alkylique d'acide gras, au moins un triglycéride ou un mélange contenant au moins un des composés cités.

5. Composition selon une des revendications 1 à 4, **caractérisée en ce que** l'ester d'acide gras ou le mélange d'esters d'acide gras comprend des acides gras saturés et/ou des acides gras insaturés.

6. Composition selon une des revendications 1 à 5, **caractérisée en ce que** l'additif particulaire inorganique présente des groupes HO-Si (groupes silanol) liés par covalence sur les particules.

7. Composition selon une des revendications 1 à 6, **caractérisée en ce que** l'additif particulaire inorganique est sélectionné parmi le groupe du dioxyde de silicium pyrogène, d'oxydes mixtes de métal-silicium pyrogènes, de la bentonite, de la montmorillonite et d'un mélange contenant au moins deux des additifs cités.

8. Composition selon une des revendications 1 à 7, **caractérisée en ce que** l'additif particulaire inorganique présente un dioxyde de silicium pyrogène avec une teneur en SiO₂ supérieure ou égale à 99,8 % en poids (de l'additif calciné) et la teneur en chlorures ainsi qu'en oxydes métalliques étrangers est inférieure ou égale à 0,2 % en poids par rapport à l'additif.

9. Composition selon une des revendications 1 à 8, **caractérisée en ce que** l'additif particulaire inorganique est du dioxyde de silicium pyrogène avec une surface spécifique d'au moins 200 m²/g.

10. Composition selon la revendication 1 ou 9, **caractérisée en ce que** l'additif particulaire inorganique est du dioxyde de silicium pyrogène avec une surface spécifique d'au moins 300 m²/g.

11. Composition selon une des revendications 1 à 10, **caractérisée en ce que** les esters d'acide gras fluides à température ambiante sont sélectionnés parmi le tri-caprylate de glycérine (tri-octoate de glycérine), le tri-caprinate de glycérine (tri-décanoate de glycérine), le dicaprylate de propylèneglycol et le dicaprate de proprylène et des mélanges contenant au moins deux des composés cités.

12. Composition selon une des revendications 1 à 11, **caractérisée en ce que** les esters d'acide gras fluides à température ambiante sont sélectionnés parmi les esters méthyliques, les esters éthyliques, les esters n-propyliques, les esters isopropyliques et les glycérides de l'acide caprylique (acide octanoïque), de l'acide caprique (acide décanoïque), de l'acide laurique (acide dodécanoïque), de l'acide myristique (acide tétradécanoïque), de l'acide palmitique (acide hexadécanoïque) et de l'acide stéarique (acide octadécanoïque) et de leurs mélanges.

13. Composition selon une des revendications 1 à 12, **caractérisée en ce que** le polymère organique est sélectionné parmi l'ester alkylique d'acide poly(alkyl-2-acrylique), l'ester alkylique d'acide poly(aryl-2-acrylique), l'ester alkylique d'acide poly(arylalkyl-2-acrylique) à chaque fois indépendamment avec 1 à 18 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans les groupes alkylester ou un mélange comprenant au moins deux des polymères cités.

14. Composition selon la revendication 1 ou 13, **caractérisée en ce que** le polymère organique comprend au moins un ester méthylique d'acide poly(méthacrylique) (PMMA) et le monomère comprend un ester méthylique d'acide méthacrylique (MMA).

15. Composition selon une des revendications 1 ou 14, **caractérisée en ce que** le polymère organique est sélectionné parmi le groupe de l'ester méthylique d'acide poly(méthacrylique), l'ester éthylique d'acide poly(méthacrylique), l'ester propylique d'acide poly(méthylméthacrylique), l'ester isopropylique d'acide poly(méthacrylique), le poly(méthylméth-acrylate-co-méthyl-acrylate), le poly(styrène-co-méthyl-méth-acrylate), des copolymères des composés cités et un mélange d'au moins deux de ces polymères.

16. Composition selon une des revendications 1 à 15, **caractérisée en ce que** le monomère est sélectionné parmi au moins un ester alkylique d'acide alkyl-2-acrylique, ester alkylique d'acide aryl-2-acrylique, ester alkylique d'acide arylalkyl-2-acrylique à chaque fois indépendamment avec 1 à 18 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans les groupes alkylester ou un mélange comprenant au moins deux des monomères cités.

17. Kit présentant une pâte A et une pâte B, dans lequel
(a) la pâte A contient
(a1) au moins un monomère polymérisable par voie radicalaire,
(a2) au moins un polymère organique soluble dans (a1), et
(a3) au moins un initiateur de polymérisation ;
(b) la pâte B contient
(b1) au moins un monomère polymérisable par voie radicalaire,
(b2) au moins un polymère organique soluble dans (b1), et
(b3) au moins un accélérateur de polymérisation ; et dans lequel au moins une des pâtes A et B comprend en tant que composante (a4) ou (b4) au moins un additif particulaire inorganique avec une surface spécifique d'au moins 40 m²/g, dans lequel l'additif présente des groupes hydroxyle liés par covalence et dans lequel au moins une des pâtes A et B présente en tant que composante (a5) ou (b5) au moins un ester d'acide gras ou un mélange d'esters d'acide gras, dans lequel l'au moins un additif particulaire inorganique est présent à 0,02 à 0,14 % pourcent en poids par rapport à la composition totale et l'au moins un ester d'acide gras ou le mélange d'esters d'acide gras est présent à 1,0 à 6,0 % pourcent en poids par rapport à la composition totale, et l'au moins un ester d'acide gras ou le mélange d'esters d'acide gras est fluide à température ambiante (18 à 25°C).

18. Kit présentant une composante pulvérulente C et une composante de monomère D, dans lequel la
(c) composante pulvérulente C contient
(c1) au moins un polyacrylate pulvérulent,
(c2) au moins un opacifiant radiographique pulvérulent, et
(c3) au moins un initiateur de polymérisation ;
(d) composante de monomère D contient
(d1) au moins un monomère polymérisable par voie radicalaire,
(d2) en option un polymère organique soluble dans (d1), et
(d3) au moins un accélérateur de polymérisation ;
et dans lequel au moins la composante pulvérulente C ou composante de monomère D comprend en tant que composante (c4) ou (d4) au moins un additif particulaire inorganique avec une surface spécifique d'au moins 40 m²/g, et dans lequel l'additif présente des groupes hydroxyle liés par covalence, et dans lequel au moins la composante de monomère D ou la composante pulvérulente C présente en tant que composante (c5) ou (d5) au moins un ester d'acide gras ou un mélange d'esters d'acide gras, dans lequel l'au moins un additif particulaire inorganique est présent à 0,02 à 0,14 % pourcent en poids par rapport à la composition totale et l'au moins un ester d'acide gras ou le mélange d'esters d'acide gras est présent à 1,0 à 6,0 % pourcent en poids par rapport à la composition totale, et l'au moins un ester d'acide gras ou le mélange d'esters d'acide gras est fluide à température ambiante (18 à 25°C).

19. Ciment osseux durcissable pouvant être obtenu par mélange des pâtes A et B contenues dans le kit selon la revendication 17 ou de la composante pulvérulente C contenue dans le kit selon la revendication 18 avec la composante de monomère D.

20. Corps moulé pouvant être obtenu par polymérisation d'une composition selon une des revendications 1 à 16 ou par mélange des pâtes A et B contenues dans le kit selon la revendication 17 ou de la composante pulvérulente C contenue dans le kit selon la revendication 18 avec la composante de monomère D et polymérisation.
